(19) Europäisches Patentamt / European Patent Office / Office européen des brevets

(11) **EP 4 261 835 A1**

(12) **EUROPEAN PATENT APPLICATION**

(43) Date of publication:
**18.10.2023 Bulletin 2023/42**

(21) Application number: **23167779.0**

(22) Date of filing: **13.04.2023**

(51) International Patent Classification (IPC):
**G16H 20/17** (2018.01)   **A61M 5/14** (2006.01)
**G16H 40/67** (2018.01)   **G16H 50/20** (2018.01)

(52) Cooperative Patent Classification (CPC):
**G16H 20/17; A61M 5/14; G16H 40/67; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.04.2022 US 202263330872 P**

(71) Applicant: **Insulet Corporation**
**Acton MA 01720 (US)**

(72) Inventors:
• **LEE, Joon Bok**
  **Acton (US)**

• **ZHENG, Yibin**
  **Hartland (US)**
• **O'CONNOR, Jason**
  **Acton (US)**
• **ZADE, Ashutosh**
  **San Diego (US)**
• **HARDY, Jonathan**
  **Reading (US)**

(74) Representative: **Peterreins Schley**
**Patent- und Rechtsanwälte PartG mbB**
**Hermann-Sack-Straße 3**
**80331 München (DE)**

(54) **SYSTEM AND METHOD FOR CREATING OR ADJUSTING MANUAL BASAL PROFILES**

(57)   Disclosed herein are various embodiments comprising methods to propose or modify a basal profile of a user, wherein the basal profile is delivered by an automatic drug delivery system operating in manual mode or is administered manually by the user. Suggestions of initial basal profiles or modifications to existing basal profiles may be based on previous glucose control outcomes or previous insulin delivery as recorded by the automatic drug delivery system.

FIG. 1

EP 4 261 835 A1

**Description**

**BACKGROUND**

Many conventional automatic drug delivery (ADD) systems are well known, including, for example, wearable drug delivery devices. The drug delivery device can be designed to deliver any type of liquid drug to a user. In specific embodiments, the drug delivery device can be, for example, an OmniPod® drug delivery device manufactured by Insulet Corporation of Acton, Massachusetts. The drug delivery device can be a drug delivery device such as those described in U.S. Pat. No. 7,303,549, U.S. Pat. No. 7,137,964, or U.S. Pat. No. 6,740,059, each of which is incorporated herein by reference in its entirety.

It is common for automatic drug delivery systems to provide basal doses of a liquid drug while the user manually provides bolus doses to compensate for meal ingestion. In some cases, the automatic drug delivery system provides the basal doses in accordance with a basal profile which specifies the timing and size of the basal doses to be delivered during that segment. The basal profile is used as an input to a medication delivery algorithm. For new users of an automatic drug delivery system, the transition from multiple daily injection therapy to insulin pump therapy is often difficult as the basal rates utilized by the automatic drug delivery system may require the determination of a significant number of parameters that are unfamiliar to the users. Further, it is desirable to be able to refine the basal profile of the user over time based on actual glucose control outcomes to ensure that the user receives safe insulin delivery, even during circumstances when glucose readings may not be available.

Therefore, it would be desirable to have the automatic drug delivery system provide suggestions for an initial basal profile for a new user of the system and, in addition, provide suggested changes to a user's basal profile based on glucose control outcomes.

**DEFINITIONS**

As used herein, the term _"liquid drug"_ should be interpreted to include any drug in liquid form capable of being administered by a drug delivery device via a subcutaneous cannula, including, for example, insulin, GLP-1, pramlintide, morphine, blood pressure medicines, chemotherapy drugs, fertility drugs, or the like, or co-formulations of two or more of GLP-1, pramlintide, and insulin.

**SUMMARY**

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In a first embodiment of the invention, an automatic drug delivery system may propose an initial basal profile that meets standard use cases of typical users that transition from multiple daily injection (MDI) therapy to insulin pump therapy. Specifically, the method takes advantage of a user's typical mode of operation using MDI therapy, that is, injection of long-acting insulin once per day, and additional insulin injections to compensate for meals throughout the day, and automatically translates this into a recommended basal profile for the user. The robustness of automatic drug delivery systems provides an increased margin of error against potential inaccuracies in the recommendations, allowing the proposed basal profiles to be safe for users.

In the second embodiment of the invention, an algorithm implemented by the automatic drug delivery system is provided to propose a basal profile comprising an overnight segment and a daytime segment based on observed glucose control outcomes. The overnight and daytime segments correspond to common life patterns of all users, that is, sleep and daily use, with the understanding that minor variations in insulin needs between the segments can be addressed by the automatic drug delivery system.

In a third embodiment of the invention, an algorithm implemented by the automatic drug delivery system is provided to propose recommended changes in the user's basal profile based on glucose control outcomes recorded by the automatic drug delivery system.

**BRIEF DESCRIPTION OF THE DRAWINGS**

In the drawings, like reference characters generally refer to the same parts throughout the different views. In the following description, various embodiments of the present invention are described with reference to the following drawings, in which:

FIG. 1 illustrates a functional block diagram of an exemplary system suitable for implementing the systems and methods disclosed herein.

**FIG. 2** is a flowchart showing a first embodiment of the invention in which a basal profile is proposed based on the current MDI parameters of the user.

**FIG. 3** is a flowchart showing a second embodiment of the invention in which a basal profile comprising an overnight segment and a daytime segment is proposed based on an assessment of a minimum mean total insulin delivery over an 8-hour period.

**FIG. 4** is a flowchart showing a third embodiment of the invention in which modifications to a user's basal profile are proposed based on unitless terms representing a mean quality of the user's glucose control for hyperglycemic and hypoglycemic incidence.

**FIGS. 5A-5C** are illustrations of user interface screen showing multiple different ways of visualizing proposed basal profiles or proposed modifications to basal profiles in a user interface of an automatic drug delivery system.

## DETAILED DESCRIPTION

This disclosure presents various systems, components and methods for moving a liquid drug from a liquid reservoir in a drug delivery device to a patient interface, such as a needle or cannula. The embodiments described herein provide one or more advantages over conventional, prior art systems, components and methods, namely, a smaller overall footprint of the drug delivery device.

Various embodiments of the present invention include systems and methods for delivering a medication to a user using a drug delivery device (sometimes referred to herein as a "pod"), either autonomously, or in accordance with a wireless signal received from an electronic device. In various embodiments, the electronic device may be a user device comprising a smartphone, a smart watch, a smart necklace, a module attached to the drug delivery device, or any other type or sort of electronic device that may be carried by the user or worn on the body of the user and that executes an algorithm that computes the times and dosages of delivery of the medication.

For example, the user device may execute an "artificial-pancreas" (AP) algorithm that computes the times and dosages of delivery of insulin. The user device may also be in communication with a sensor, such as a glucose sensor or a continuous glucose monitor (CGM), that collects data on a physical attribute or condition of the user, such as a glucose level. The sensor may be disposed in or on the body of the user and may be part of the drug delivery device or may be a separate device.

Alternatively, the drug delivery device may be in communication with the sensor in lieu of or in addition to the communication between the sensor and the user device. The communication may be direct (if, e.g., the sensor is integrated with or otherwise a part of the drug delivery device) or remote/wireless (if, e.g., the sensor is disposed in a different housing than the drug delivery device). In these embodiments, the drug delivery device contains computing hardware (e.g., a processor, memory, firmware, etc.) that executes some or all of the algorithm that computes the times and dosages of delivery of the medication.

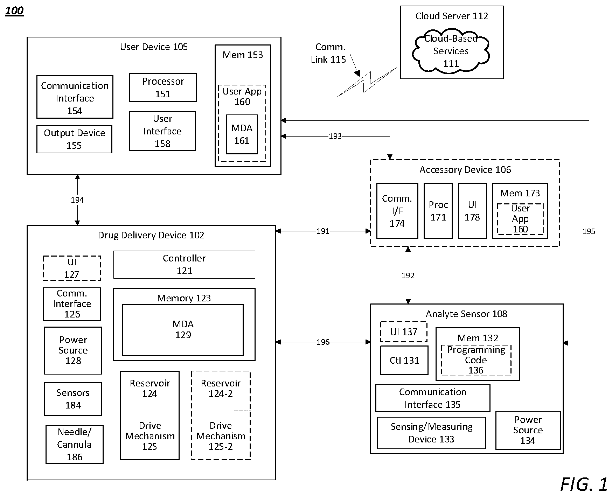

**FIG. 1** illustrates a functional block diagram of an exemplary drug delivery system 100 suitable for implementing the systems and methods described herein. The drug delivery system 100 may implement (and/or provide functionality for) a medication delivery algorithm, such as an artificial pancreas (AP) application, to govern or control the automated delivery of a drug or medication, such as insulin, to a user (e.g., to maintain euglycemia - a normal level of glucose in the blood). The drug delivery system 100 may be an automated drug delivery system that may include a drug delivery device 102 (which may be wearable), an analyte sensor 108 (which may also be wearable), and a user device 105.

Drug delivery system 100, in an optional example, may also include an accessory device 106, such as a smartwatch, a personal assistant device, a smart insulin pen, or the like, which may communicate with the other components of system 100 via either a wired or wireless communication links 191-193.

*User Device*

The user device 105 may be a computing device such as a smartphone, a smartwatch, a tablet, a personal diabetes management (PDM) device, a dedicated diabetes therapy management device, or the like. In an example, user device 105 may include a processor 151, device memory 153, a user interface 158, and a communication interface 154. The user device 105 may also contain analog and/or digital circuitry that may be implemented as a processor 151 for executing processes based on programming code stored in device memory 153, such as user application 160 incorporating medication delivery algorithm (MDA) 161 to manage a user's blood glucose levels and for controlling the delivery of the drug, medication, or therapeutic agent to the user, as well for providing other functions, such as calculating carbohydrate-compensation dosage, a correction bolus dosage and the like as discussed below. The user device 105 may be used to activate, deactivate, trigger a needle/canula insertion, program, adjust settings, and/or control operation of drug delivery device 102 and/or the analyte sensor 103 as well as the optional smart accessory device 106.

The processor 151 may also be configured to execute programming code stored in device memory 153, such as the user app 160. The user app 160 may be a computer application that is operable to deliver a drug based on information received from the analyte sensor 103, the cloud-based services 111 and/or the user device 105 or optional accessory device 106. The memory 153 may also store programming code to, for example, operate the user interface 158 (e.g., a touchscreen device, a camera or the like), the communication interface 154 and the like. The processor 151, when executing user app 160, may be configured to implement indications and notifications related to meal ingestion, blood glucose measurements, and the like. The user interface 158 may be under the control of the processor 151 and be configured to present a graphical user interface that enables the input of a meal announcement, adjust setting selections and the like as described herein.

In a specific example, when the user app 160 includes MDA 161, the processor 151 is also configured to execute a diabetes treatment plan (which may be stored in a memory) that is managed by user app 160. In addition to the functions mentioned above, when user app 160 is an AP application, it may further provide functionality to determine a carbohydrate-compensation dosage, a correction bolus dosage and determine a real-time basal dosage according to a diabetes treatment plan. In addition, as a MDA 161, user app 160 provides functionality to output signals to the drug delivery device 102 via communications interface 154 to deliver the determined bolus and/or basal dosages.

The communication interface 154 may include one or more transceivers that operate according to one or more radio-frequency protocols. In one embodiment, the transceivers may comprise a cellular transceiver and a Bluetooth® transceiver. The communication interface 154 may be configured to receive and transmit signals containing information usable by user app 160.

User device 105 may be further provided with one or more output devices 155 which may be, for example, a speaker or a vibration transducer, to provide various signals to the user.

*Drug Delivery Device*

In various exemplary embodiments, drug delivery device 102 may include a reservoir 124 and drive mechanism 125, which are controllable by controller 121, executing a medication delivery algorithm (MDA) 129 stored in memory 123, which may perform some or all of the functions of the AP application described above, such that user device 105 may be unnecessary for drug delivery device 102 to carry out drug delivery and control. Alternatively, controller 121 may act to control reservoir 124 and drive mechanism 125 based on signals received from user app 160 executing on a user device 105 and communicated to drug delivery device 102 via communication link 194. Drive mechanism 125 may operate to longitudinally translate a plunger through the reservoir, so as to force the liquid drug through an outlet fluid port to needle / cannula 186. Alternatively, other types of drive mechanisms may be used.

In an alternate embodiment, drug delivery device 102 may also include an optional second reservoir 124-2 and second drive mechanism 125-2 which enables the independent delivery of two different liquid drugs. As an example, reservoir 124 may be filled with insulin, while reservoir 124-2 may be filled with glucagon, or Pramlintide, or GLP-1. In some embodiments, each of reservoirs 124, 124-2 may be configured with a separate drive mechanism 125, 125-2, respectively, which may be separately controllable by controller 121 under the direction of MDA 129. Both reservoirs 124, 124-2 may be connected to a common needle / cannula 186.

Drug delivery device 102 may be optionally configured with a user interface 127 providing a means for receiving input from the user and a means for outputting information to the user. User interface 127 may include, for example, light-emitting diodes, buttons on a housing of drug delivery device 102, a sound transducer, a micro-display, a microphone, an accelerometer for detecting motions of the device or user gestures (e.g., tapping on a housing of the device) or any other type of interface device that is configured to allow a user to enter information and/or allow drug delivery device 102 to output information for presentation to the user (e.g., alarm signals or the like).

Drug delivery device 102 includes a patient interface 186 for interfacing with the user to deliver the liquid drug. Patient interface may be, for example, a needle or cannula for delivering the drug into the body of the user (which may be done subcutaneously, intraperitoneally, or intravenously). Drug delivery device 102 may further include a mechanism for inserting the needle / cannula 186 into the body of the user, which may be integral with or attachable to drug delivery device 102. The insertion mechanism may comprise, in one embodiment, an actuator that inserts the needle / cannula 186 under the skin of the user and thereafter retracts the needle, leaving the cannula in place. The actuator may be triggered by user device 105 or may be a manual firing mechanism comprising springs or other energy storing mechanism, that causes the needle / cannula 186 to penetrate the skin of the user.

In one embodiment, drug delivery device 102 includes a communication interface 126, which may be a transceiver that operates according to one or more radio-frequency protocols, such as Bluetooth®, Wi-Fi, near-field communication, cellular, or the like. The controller 121 may, for example, communicate with user device 105 and an analyte sensor 108 via the communication interface 126.

In some embodiments, drug delivery device 102 may be provided with one or more sensors 184. The sensors 184 may include one or more of a pressure sensor, a power sensor, or the like that are communicatively coupled to the controller 121 and provide various signals. For example, a pressure sensor may be configured to provide an indication of the fluid pressure detected in a fluid pathway between the patient interface 186 and reservoir 124. The pressure sensor may be coupled to or integral with the actuator for inserting the patient interface 186 into the user. In an example, the controller 121 may be operable to determine a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount. In one embodiment, analyte sensor 108 may be integral with drug delivery device 102.

Drug delivery device 102 further includes a power source 128, such as a battery, a piezoelectric device, an energy harvesting device, or the like, for supplying electrical power to controller 121, memory 123, drive mechanisms 125 and/or other components of drug delivery device 102.

Drug delivery device 102 may be configured to perform and execute processes required to deliver doses of the medication to the user without input from the user device 105 or the optional accessory device 106. As explained in more detail, MDA 129 may be operable, for example, to determine an amount of insulin to be delivered, IOB, insulin remaining, and the like and to cause controller 121 to activate drive mechanism 125 to deliver the medication from reservoir 124. MDA 129 may take as input data received from the analyte sensor 108 or from user app 160.

The reservoirs 124, 124-2 may be configured to store drugs, medications or therapeutic agents suitable for automated delivery, such as insulin, Pramlintide, GLP-1, co-formulations of insulin and GLP-1, glucagon, morphine, blood pressure medicines, arthritis drugs, chemotherapy drugs, fertility drugs, hormonal drugs, or the like.

Drug delivery device 102 may be a wearable device and may be attached to the body of a user, such as a patient or diabetic, at an attachment location and may deliver any therapeutic agent, including any drug or medicine, such as insulin or the like, to a user at or around the attachment location. A surface of drug delivery device 102 may include an adhesive to facilitate attachment to the skin of a user.

When configured to communicate with an external device, such as the user device 105 or the analyte sensor 108, drug delivery device 102 may receive signals over the wired or wireless link 194 from the user device 105 or from the analyte sensor 108. The controller 121 of drug delivery device 102 may receive and process the signals from the respective external devices as well as implementing delivery of a drug to the user according to a diabetes treatment plan or other drug delivery regimen.

*Accessory Device*

Optional accessory device 106 may be, a wearable smart device, for example, a smart watch (e.g., an Apple Watch®), smart eyeglasses, smart jewelry, a global positioning system-enabled wearable, a wearable fitness device, smart clothing, or the like. Accessory device 106 may alternatively be a smart insulin pen that works with drug delivery device 102 in managing blood glucose and treating diabetes of a user. Similar to user device 105, the accessory device 106 may also be configured to perform various functions including controlling or communicating with drug delivery device 102. For example, the accessory device 106 may include a communication interface 174, a processor 171, a user interface 178 and a memory 173. The user interface 178 may be a graphical user interface presented on a touchscreen display of the smart accessory device 107. The memory 173 may store programming code to operate different functions of the smart accessory device 107 as well as an instance of the user app 160, or a pared-down version of user app 160 with reduced functionality. In some instances, accessory device 107 may also include sensors of various types.

*Analyte Sensor*

The analyte sensor 108 may include a controller 131, a memory 132, a sensing/measuring device 133, an optional user interface 137, a power source/energy harvesting circuitry 134, and a communication interface 135. The analyte sensor 108 may be communicatively coupled to the processor 151 of the management device 105 or controller 121 of drug delivery device 102. The memory 132 may be configured to store information and programming code 136.

The analyte sensor 108 may be configured to detect one or multiple different analytes, such as glucose, lactate, ketones, uric acid, sodium, potassium, alcohol levels or the like, and output results of the detections, such as measurement values or the like. The analyte sensor 108 may, in an exemplary embodiment, be configured as a continuous glucose monitor (CGM) to measure a blood glucose values at a predetermined time interval, such as every 5 minutes, every 1 minute, or the like. The communication interface 135 of analyte sensor 108 may have circuitry that operates as a transceiver for communicating the measured blood glucose values to the user device 105 over a wireless link 195 or with drug delivery device 102 over the wireless communication link 108. While referred to herein as an analyte sensor 108, the sensing/measuring device 133 of the analyte sensor 108 may include one or more additional sensing elements, such as a glucose measurement element, a heart rate monitor, a pressure sensor, or the like. The controller 131 may include discrete, specialized logic and/or components, an application-specific integrated circuit, a microcontroller or processor that executes software instructions, firmware, programming instructions stored in memory (such as memory 132), or any combination thereof.

Similar to the controller 121 of drug delivery device 102, the controller 131 of the analyte sensor 108 may be operable to perform many functions. For example, the controller 131 may be configured by programming code 136 to manage the collection and analysis of data detected by the sensing and measuring device 133.

Although the analyte sensor 108 is depicted in **FIG. 1** as separate from drug delivery device 102, in various embodiments, the analyte sensor 108 and drug delivery device 102 may be incorporated into the same unit. That is, in various examples, the analyte sensor 108 may be a part of and integral with drug delivery device 102 and contained within the same housing as drug delivery device 102 or an attachable housing thereto. In such an example configuration, the controller 121 may be able to implement the functions required for the proper delivery of the medication alone without any external inputs from user device 105, the cloud-based services 111, another sensor (not shown), the optional accessory device 106, or the like.

*Cloud-Based Services*

Drug delivery system 100 may communicate with or receive services from a cloud server 122 providing cloud-based services 111. Services provided by cloud server 112 may include data storage that stores personal or anonymized data, such as blood glucose measurement values, historical IOB or TDI, prior carbohydrate-compensation dosage, and other forms of data. In addition, the cloud-based services 111 may process anonymized data from multiple users to provide generalized information related to TDI, insulin sensitivity, IOB and the like. The communication link 115 that couples the cloud server 112 to other components of system 100, for example, devices 102, 105, 106, 108 of system 100 may be a cellular link, a Wi-Fi link, a Bluetooth® link, or a combination thereof.

*Communication Links*

The wireless communication links 115 and 191-196 may be any type of wireless link operating using known wireless communication standards or proprietary standards. As an example, the wireless communication links 191-196 may provide communication links based on Bluetooth®, Zigbee®, Wi-Fi, a near-field communication standard, a cellular standard, or any other wireless protocol via the respective communication interfaces 126, 135, 154 and 174.

*Operational Example*

In an operational example, user application 160 implements a graphical user interface that is the primary interface with the user and is used to activate drug delivery device 102, trigger a needle/cannula insertion, start and stop drug delivery device 102, program basal and bolus calculator settings for manual mode as well as program settings specific for automated mode (hybrid closed-loop or closed-loop).

User app 160, provides a graphical user interface 158 that allows for the use of large text, graphics, and on-screen instructions to prompt the user through the set-up processes and the use of system 100. It may also be used to program the user's custom basal insulin delivery profile, accept a recommended basal insulin delivery profile, check the status of drug delivery device 102, initiate bolus doses of insulin, make changes to a patient's insulin delivery profile, handle system alerts and alarms, or allow the user to switch between automated mode and manual mode.

User app 160 may be configured to operate in a manual mode in which user app 160 will deliver insulin at programmed basal rates and user-defined bolus amounts with the option to set temporary basal profiles. The controller 121 will also have the ability to function as a sensor-augmented pump in manual mode, using sensor glucose data provided by the analyte sensor 108 to populate the bolus calculator.

User app 160 may be configured to operate in an automated mode in which user app 160 supports the use of one or multiple target blood glucose values that may be adjusted manually or automatically by the system. For example, in one embodiment, target blood glucose values can range from 110-150 mg/dL, in 10 mg/dL increments, in 5 mg/dL increments, or other increments, but preferably 10 mg/dL increments. The experience for the user will reflect current setup flows whereby the healthcare provider assists the user to program basal rates, glucose targets and bolus calculator settings. These in turn will inform the user app 160 for insulin dosing parameters. The insulin dosing parameters will be adapted over time based on the total daily insulin (TDI) delivered during each use of drug delivery device 102. A temporary hypoglycemia protection mode may be implemented by the user for various time durations in automated mode. With hypoglycemia protection mode, the algorithm reduces insulin delivery and is intended for use over temporary durations when insulin sensitivity is expected to be higher, such as during exercise or fasting.

The user app 160 (or MDA 129) may provide periodic insulin micro-boluses based upon past glucose measurements and/or a predicted glucose over a prediction horizon (e.g., 60 minutes). Optimal post-prandial control may require the user to give meal boluses in the same manner as current pump therapy, but normal operation of the user app 160 will compensate for missed meal boluses and mitigate prolonged hyperglycemia. The user app 160 uses a control-to-target strategy that attempts to achieve and maintain a set target glucose value, thereby reducing the duration of prolonged hyperglycemia and hypoglycemia.

In some embodiments, user device 105 and the analyte sensor 108 may not communicate directly with one another. Instead, data (e.g., blood glucose readings) from analyte sensor may be communicated to drug delivery device 102 via link 196 and then relayed to user device 105 via link 194. In some embodiments, to enable communication between analyte sensor 108 and user device 105, the serial number of the analyte sensor must be entered into user app 160.

User app 160 may provide the ability to calculate a suggested bolus dose through the use of a bolus calculator. The bolus calculator is provided as a convenience to the user to aid in determining the suggested bolus dose based on ingested carbohydrates, most-recent blood glucose readings (or a blood glucose reading if using fingerstick), programmable correction factor, insulin to carbohydrate ratio, target glucose value, and insulin on board (IOB). IOB is estimated by user app 160 taking into account any manual bolus and insulin delivered by the algorithm.

*Description of Embodiments*

In a first embodiment of the invention, a method is provided to initialize MDA 129, 161 with a basal profile for use in a manual mode of system 100 and an initial total daily insulin (TDI) quantity for use in an automatic mode of system 100.

The proposed basal profile and TDI are based on parameters of the user's multiple daily injection (MDI) therapy to allow the user to easily transition from MDI-based therapy to therapy based on the automated drug delivery system. The method proposes an initial basal profile that meets standard use cases of typical users transitioning from MDI therapy to use of an automatic drug delivery system. The method will be explained in the context of a user having parameters for MDI therapy as shown in Table 1.

Table 1

| MDI Therapy Parameters | |
|---|---|
| Long-Acting Insulin | Short-Acting Insulin |
| 15U at 19:00 | 5U at 7:00 |
| | 7U at 13:00 |
| | 3U at 20:00 |
| | ... |

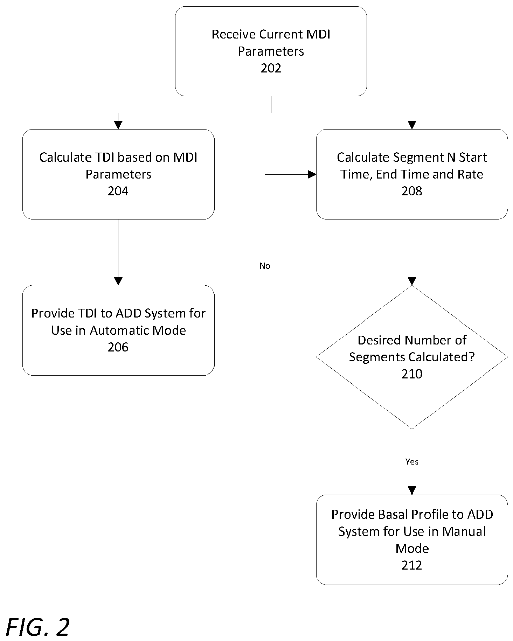

Under typical use cases, users would need to work with an endocrinologist or other healthcare professionals to translate the MDI parameters shown in Table 1 into an associated basal profile before utilizing an automated drug delivery system, for example, system 100 described above and shown in **FIG. 1,** to provide continuous subcutaneous insulin infusion via a pump. However, when these parameters are utilized with an automatic drug delivery system of the type described herein, logic incorporating typical physician-guided heuristics can be utilized to provide a reasonable starting point for initial clinical basal profiles, which can then be adapted by system 100 over time. In this embodiment of the invention, system 100 can request the MDI therapy parameters as inputs and translate them automatically into an initial total daily insulin dose for use in automatic mode and a basal profile for use in manual mode, in accordance with the method shown in flowchart form in **FIG. 2.** For example, a user may be able to enter via a user interface the time and the number of units of long-acting or short-acting insulin that the user typically delivers via MDI.

In a first step 202 of the method, the current MDI parameters, for example, those shown in Table 1, are received. In one embodiment, the current MDI parameters may be entered into user app 160 by the user via user interface 158 on user device 105. In other embodiments, the user may enter the MDI parameters via a cloud-based service 111 and the cloud-based service 111 may download the parameters to user app 160 via communication link 115.

In a second step 204, the user's total daily insulin, based on the MDI parameters, may be calculated in accordance with Eq. (1):

$$TDI_{AID} = X\left(LA + \sum_{i=1}^{N} SA_i\right)$$

$$(1)$$

where:

LA is the user's total daily long-acting insulin;
$SA_i$ is the user's short-acting insulin from daily segment *i*;
N is the total number of daily segments in the user's MDI profile; and
X is a tunable parameter can vary between approximately 0.7 and approximately 1.0, or more preferably between approximately 0.8 and approximately 0.9, and represents an optional reasonable reduction of the total insulin specified by the MDI parameters as a starting point for the automatic drug delivery system when operated in automatic mode. Typically, a user switching from MDI therapy to therapy via an automatic drug delivery system does not require as much insulin due to the better efficiency and diabetes control of an automated system. The percent of insulin needed is typically about 10% to 20% less than the user was administering during MDI therapy.

The resulting TDI value can be used as a reasonable starting point for the MDA of an automatic drug delivery system to begin insulin delivery in automatic mode without significant risks of over-delivery or under-delivery, and without a period of conservative delivery for initial adaptivity. At step 206, the TDI value is provided to MDA 129, 161 of system 100 for use as a basis for insulin delivery in automatic mode.

The user's current MDI parameters can also be used a formulate a baseline basal profile that can be used by MDA 129,161 when in manual mode. That is, the insulin delivery in manual mode is performed in accordance with the basal profile and not as the result of any algorithmic calculations. This may be especially valuable in instances wherein CGM readings of the user are unavailable to MDA 129, 161.

At the loop formed by steps 208 and 210, the start time, end time and rate (e.g., units/h) of each of the segments of the user's basal profile during periods of insulin delivery in manual mode can be reasonably initialized. In one exemplary embodiment, the system proposes a basal profile having two segments, one segment designed for periods where there is the least amount of meal activity, that is, ~12 hours away from the mean time when short-acting insulin is delivered, and another segment for the periods of meals. As the length of one day is 12 hours, applying this 12 hour offset from the mean meal ingestion time allows the resulting segment to be furthest opposing the average periods of meal ingestion in one day. The duration, represented by the variable "Q" in the equations below, may be a tunable parameter, for example, 8 hours, and can vary between, for example, 6 hours and 10 hours. The variable "Q" represents a period of reduced insulin delivery, for example, times when the user is not typically bolusing with short-acting insulin. In this exemplary embodiment, the start time of the first segment can be specified as:

$$\frac{\sum_{i=1}^{N} T_{SA,i}}{N} + 12$$

where $T_{SA,i}$ is the average time that short-acting insulin was delivered during MDI therapy. The end time of the first segment can be specified as:

$$\frac{\sum_{i=1}^{N} T_{SA,i}}{N} + 12 + Q$$

For the second segment, the start time is simply the end time of the first segment, and the end time of the second segment is the same as the start time of the first segment for the next day.

These segments can be determined in other ways. For example, using an accelerometer or other sensor on the drug delivery device 102, the system may determine when the user is at rest or sleeping. In this manner, the user's sleeping hours may be determined, which may be carried over into when the user is not typically bolusing with short-acting insulin or is not consuming food.

For the first segment, the basal profile rate can be specified, for example, as:

$$A\left(X \cdot \frac{LA}{24}\right) U/h$$

while the basal profile rate for the second segment can be specified as:

$$\frac{1 - A\frac{Q}{24}}{1 - \frac{Q}{24}}\left(X\frac{LA}{24}\right) U/h$$

Here, A represents the relative reduction in the user's basal insulin needs during periods without meal ingestion, with typical values being 0.8, or a 20% reduction compared to average basal needs, but which may typically vary between 0.5 and 0.95, or similar values. The formulation defined in these equations allow a corresponding increase in basal insulin delivery for the second segment, while maintaining the same total insulin delivery as originally defined in equation (1).

Typically, these periods may indicate times of reduced meal activity, and, thus, the associated insulin delivery rate (basal profile rate for the first segment) can be considered to benefit from a reduction versus the typical hourly basal rate, such as by -20%, which may be a tunable parameter. Conversely, the basal delivery quantity during the remaining segment can be tuned such that the total insulin delivery still matches the user's basal insulin needs, based on the duration of the reduced insulin delivery period selected ("Q"). In the exemplary embodiment shown above having a duration of 8 hours with an insulin reduction of -20%, the resulting second basal profile would recommend an insulin increase of +10% during the longer segment of 16 hours.

In a final step 212 of the method, the basal profile is complete and provided to system 100 for used by MDA 129, 161 to guide insulin delivery when MDA 129, 161 is operating in manual mode. It should be noted that when the system is operating in automated mode, the system does not need to rely on basal profiles.

As would be realized by one of skill in the art, the various assumptions made and incorporated into the equations above are exemplary in nature only and, in variations of this embodiment, may be changed to provide different assumptions. Additionally, variations of this embodiment wherein more than two segments are calculated are contemplated to be within the scope of the invention. Further, although described for use by system 100, as would be realized by one of skill in the art, the method can be used with any automatic drug delivery system.

In a second embodiment of the invention, a basal profile for use in manual mode may be created or modified to propose an overnight basal profile segment and a daytime segment based on insulin delivery outcomes from an automated mode of system 100. This embodiment of the invention discloses a method to suggest a basal profile that identifies a common set of two segments corresponding to the common sleep and daily activity patterns of all users. Users of an automated drug delivery system and, in particular, users with type 1 diabetes, typically incorporate a period of time when they are asleep and are not ingesting any carbohydrates, with the remainder of the day characterized by being awake and ingesting carbohydrates from time to time. Although the starting and ending points of segmentation of the day may be different across users, the existence of such a segmentation can be considered one of the most generalizable facts of daily diabetic care. Further, the duration of the segment, in some embodiments, can be considered to last approximately 8 hours, or proximally 1/3 of each day, although the start and end times of the sleep segment may vary on a per user basis. Nevertheless, all users will have a period during which is a high confidence that the user will have less insulin needs due to a lower probability of carbohydrate ingestion and a higher preference for avoidance of hypoglycemia as opposed to hyperglycemia. Further, insulin delivery by system 100 typically responds more gradually to variations in the user's insulin needs compared to the user's manual insulin deliveries.

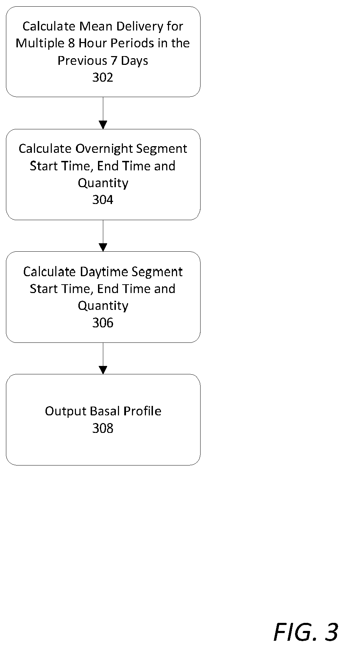

A flowchart of the second embodiment of the invention is shown in FIG. 3. The insulin delivery records of system 100 can be used to identify a customized eight hour segment during which the user's insulin needs are lower than the remaining 16 hours. Specifically, the mean total insulin delivery for any eight hour period in the previous 7 days can be calculated at step 302 of the method using Eq. (2):

$$I_{on,j} = \frac{1}{7}\sum_{k=1}^{7}\sum_{i=j}^{j+96} I_k(i); \quad j = 1 \dots 288$$

$$(2)$$

where:

$I_{on,j}$ represents the mean total insulin delivery over 8 hours starting from the $j^{th}$ cycle of each day in the previous seven days;

288 represents the number of 5-minute segments in 24 hours, but this number can be tunable;

and 96 represents the number of 5-minute segments in the 8 hour period.

The resulting 288 values of $I_{on,j}$ can then be assessed to determine the index $j_{min}$ that shows the minimum total insulin delivery. Note that this example assumes 5-minute cycles. For embodiments using other cycle intervals, Eq. (2) and the equations below will necessarily change to reflect the different cycle intervals. The $j_{min}$ index can be utilized to recommend the start time, the end time and the quantity for both the overnight and daytime segments. The overnight parameters can be calculated in step 304 of the method as follows:

$$Overnight_{start} = j_{min}$$

$$Overnight_{end} = j_{min} + 96$$

$$Overnight_{quantity} = \frac{\frac{1}{7}\sum_{k=1}^{7}\sum_{i=j_{min}}^{j_{min}+96} I_{k,AID}(i)}{8}$$

Again, these equations assume 5-minute cycles (e.g., 96 5-minute intervals equals 8 hours) and an 8-hour sleep interval. The daytime parameters can be calculated in step 306 of the method as follows:

$$Daytime_{start} = j_{min} + 96$$

$$Daytime_{end} = j_{min} + 288$$

$$Datime_{quantity} = \frac{\frac{1}{7}\sum_{k=1}^{7}\sum_{i=j_{min}+96}^{j_{min}+288} I_{k,AID}(i)}{16}$$

The overnight and daytime segments can be utilized by system 100 for improved control outcomes when used in combination with manual basal profiles. In alternate embodiments, wherein system 100 is not used, the segments can be used as manual basal profiles. Lastly, the segments can be used as an initial recommendation of basal profiles (as with the first embodiment of the invention) which can then be further adjusted by the user or the physician as needed. The end product 2-segment basal profile is output at step 308 of the method to system 100 and may be visualized in a user interface 158 of user device 105 by user app 160. It is important to note that the length of the basal segment to support the overnight period may vary, and is a tunable parameter - the value 96 in previous embodiments can be varied based on differing assumptions of the length of the overnight period, such as 6 hours (72 cycles) or 10 hours (120 cycles). This will also correspondingly vary the 16 hours of relative daytime segments to 18 (6 hours of overnight) or 14 (10 hours of overnight).

As would be recognized by one of skill in the art, the five-minute time interval used in the above equations can be modified to any time interval. Further, the equations above assume an 8 hour overnight and a 16 hour daytime segment. This can be modified to include overnight and daytime segments of any duration. Further, the second embodiment, while described as being used by system 100, may be used with any automatic drug delivery system.

In a third embodiment of the invention, an algorithm of system 100 recommends changes in the user's basal profile settings to be used in manual mode based on glucose control outcomes observed by system 100. The algorithm may be, for example, implemented as part of user app 160 or MDA 129, 161. It is important to continue to improve the user's manual basal profile settings to ensure that the user receives safe insulin delivery, especially during circumstances when blood glucose readings may not be available. In cases where the basal profile settings are input to system 100, an improved basal profile may also improve the user's glucose control performance.

In various, the user's basal profile may be one of multiple inputs that can be provided to MDA 129, 161. The result of glucose control outcomes by system 100 ($G_k(i)$ in the equations below) across the previous 7 days, can therefore be assessed for each basal profile segment $b_n$ as described below.

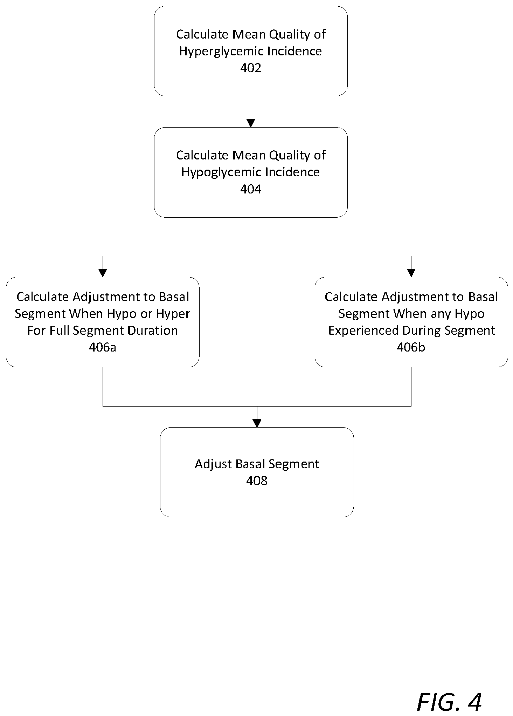

A flowchart showing the method is shown in **FIG. 4.** In first and second steps 402, 404 of the method of this embodiment, two unitless terms representing the mean quality of the user's glucose control for the $n^{th}$ basal segment for hyperglycemic and hypoglycemic incidence are calculated using Eq. (3) and Eq. (4), respectively.

$$Q_{n,hyper} = \frac{1}{7} \sum_{k=1}^{7} \frac{\sum_{i=t_{n,1}}^{t_{n,f}} max(0, G_k(i) - 180)^2}{t_{n,f} - t_{n,1}}$$

(3)

$$Q_{n,hypo} = \frac{1}{7} \sum_{k=1}^{7} \frac{\sum_{i=t_{n,1}}^{t_{n,f}} min\left(0, 70 - G_k(i)\right)^2}{t_{n,f} - t_{n,1}}$$

(4)

where:

$t_{n,1}$ is the control cycle representing the beginning of the user's $n^{th}$ basal segment; and
$t_{n,f}$ is the control cycle representing the end of the user's $n^{th}$ basal segment.

Once the quality terms have been established, the user's basal segment can be adjusted, in one variation of this embodiment, at step 406a, using Eq. (5), with the quality terms acting as a weighting factor to increase or decrease the rate of insulin delivery for the user's $n^{th}$ basal segment:

$$b_{n,new} = min\left(max\left(\left(1 - \frac{Q_{n,hypo}}{900} + \frac{Q_{n,hyper}}{48400}\right), 0.8\right), 1.2\right)$$

(5)

where:
constants 900 and 48400 are terms representing the quality terms if the user experienced the full duration of the basal segment with glucose concentrations lower than 40 mg/dL or higher than 400 mg/dL, respectively, calculating the differences against the thresholds for hypo- and hyperglycemia (70 mg/dL and 180 mg/dL); 900 represents the squared difference between 70 and 40 mg/dL, and 48400 represents the squared difference between 400 and 180 mg/dL. As would be realized, any other glucose concentrations may be used by substituting the constants.

In another variation of this embodiment, shown in step 406b, the quality terms can also be scaled such that if there was *any* incidence of hypoglycemia in the previous seven days, the basal segment would be adjusted by the severity and duration of those incidents, without consideration of hyperglycemic risk, as provided by Eq. (6):

$$b_{n,new} = \begin{cases} Q_{n,hypo} > 0 & max\left(\left(1 - \dfrac{Q_{n,hypo}}{900}\right), 0.8\right) \\[2em] Q_{n,hypo} = 0 & min\left(\left(1 + \dfrac{Q_{n,hyper}}{48400}\right), 1.2\right) \end{cases}$$

$$(6)$$

In either of the variations shown in steps 406a, 406b, the maximum and minimum adjustments may be limited to 20% in either direction, as indicated by the constants (0.8 and 1.2) in Eqs, (5) and (6), and the deviations are asymmetrically weighted based on the differences in the ranges of hyperglycemic and hypoglycemic glucose concentration readings. As would be realized, other limitations on the adjustments may be used.

At step 408, the $n^{th}$ basal segment is adjusted using the calculated adjustments from steps 406a or 406b.

It should be noted that, while this approach can be executed following control outcomes from system 100, similar assessments can also be made following manual mode control outcomes, as a history of glucose control can be applied in a generic manner in both cases. This embodiment may be used with automatic drug delivery systems other than system 100.

Any of the previously discussed embodiments of the invention may result in a change to the basal profile of the user or a proposed basal profile. In various embodiments, user app 160 may utilize user interface 158 of user device 105 to visualize basal profiles for the user.

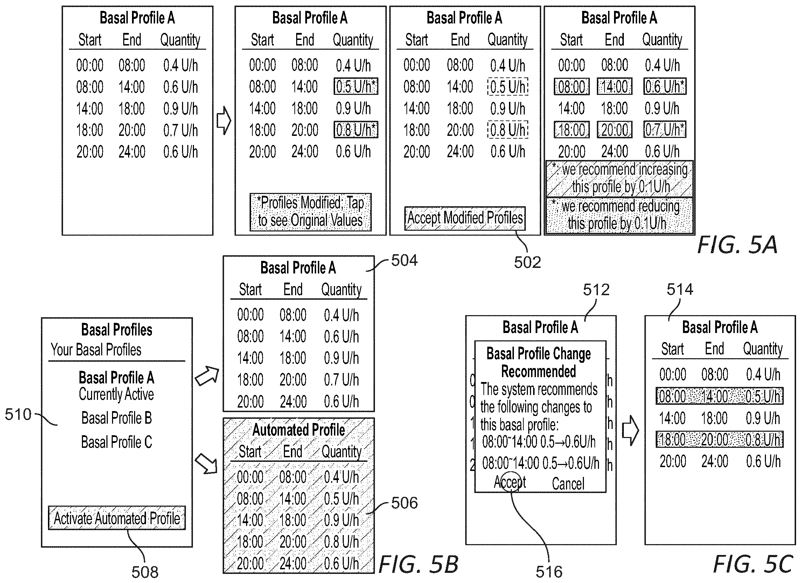

In one embodiment, shown in **FIG. 5A,** the user is shown a direct modification of the user's manual basal profiles, with different formatted highlights. In this embodiment, whenever a modified parameter is recommended, this modification can simply be formatted in a different, recognizable manner, such as by changing the color, using strikethrough, underline, highlights, or other visualizations to show the changes. Such modifications can be shown and implemented as different font colors, sizes, highlights, soft buttons or dynamic formatting (e.g., tap to confirm and/or apply). The user may be provided with a button 502 that, when selected, indicates to user app 160 that the user wishes to accept the recommended profile modifications.

In another embodiment, shown in **FIG. 5B,** the user may be shown a list 510 of basal profiles which may be selected, with at least one of the basal profiles indicated as a currently active basal profile. Once selected, the selected basal profile 504 is displayed and a proposed profile 506, which may be based on the currently selected profile 504 is also displayed. The user may be provided with a button 508 to activate profile 506.

In yet another embodiment, such as depicted in **FIG. 5C,** a user may be shown a comparison between an existing basal profile 512 and a recommended modified basal profile 514. Recommended changes to the basal profile 512 may be displayed at 514. The user may be provided with a button 516 to accept or reject the recommended changes.

As would be realized by one of skill in the art, many methods of displaying proposed basal profiles or recommended changes to a basal profile are possible and are contemplated to be within the scope of this invention.

The following examples pertain to various embodiments of the systems and methods disclosed herein for implementation of an automatic drug delivery system having a double reservoir pumping mechanism.

Example 1 is a first embodiment of the invention comprising a method of providing an initial basal profile for a user comprising receiving a set of parameters reflecting multiple daily injection therapy for the user and determining two or more segments for the basal profile, each segment comprising a start time, an end time and a delivery rate, based on the parameters.

Example 2 is an extension of Example 1, or any other example disclosed herein, wherein the parameters reflect intake of both long-acting in short-acting insulin during multiple daily injection therapy.

Example 3 is an extension of Example 2, or any other example disclosed herein, wherein the basal profile comprises two segments, one segment for periods of least meal activity and one for periods of most meal activity.

Example 4 is an extension of Example 3, or any other example disclosed herein, the method further comprising calculating a mean time for the user's intake of short-acting insulin during multiple daily injection therapy and determining a start time for the segment for periods of least meal activity as a time 12 hours after the mean time.

Example 5 is an extension of Example 4, or any other example disclosed herein, the method further comprising determining a start time for the segment for periods of most meal activity as an offset from the start time of the segment for periods of least meal activity.

Example 6 is an extension of Example 5, or any other example disclosed herein, wherein the offset varies between 6 and 10 hours.

Example 7 is an extension of Example 6, or any other example disclosed herein, the method further comprising calculating a basal rate for the segment for periods of least meal activity and calculating a basal rate for the segment for periods of most meal activity, wherein the basal rates are calculated by determining an hourly quantity of long-acting insulin based on the parameters, reducing the hourly quantity by a predetermined percentage for the segment for periods of least meal activity and increasing the hourly quantity by a predetermined percentage for the segment for periods of most meal activity.

Example 8 is an extension of Example 7, or any other example disclosed herein, wherein the hourly quantity of long-acting insulin is reduced by reduction factor reflecting a reduced insulin need when using the automatic drug delivery system.

Example 9 is an extension of Example 7, or any other example disclosed herein, wherein the reduction factor varies between 0.8 and 0.9.

Example 10 is an extension of Example 7, or any other example disclosed herein, wherein for the segment for periods of least meal activity is reduced by proximally 20% and wherein the increase quantity for the segment for periods of most meal activity is increased by approximately 10%.

Example 11 is an extension of Example 8, or any other example disclosed herein, the method further comprising providing the basal profile to an automatic drug delivery system for use in manual mode.

Example 12 is an extension of Example 11, or any other example disclosed herein, the method further comprising visualizing the basal profile in a user interface of the automatic drug delivery system.

Example 13 is a second embodiment of the invention comprising a method of providing an initial total daily insulin input for an automatic drug delivery system operating in automatic mode comprising receiving a set of parameters reflecting on multiple daily injection therapy for the user, calculating the total data insulin administered to the user based on the parameters, reducing the total daily insulin by reduction factor reflecting a reduced insulin need and providing the adjusted total daily insulin to the automatic drug delivery system as a starting basis for operation in automatic mode.

Example 14 is an extension of example 13, or any other example disclosed herein, wherein the reduction factor varies between 0.8 and 0.9.

Example 15 is a third embodiment of the invention comprising a method of providing a basal profile for a user comprising an overnight segment and a daytime segment, the method comprising determining a mean total insulin delivery for any daily period over predetermined past number of days, determining the daily period having the lowest mean total insulin delivery, beginning the overnight segment at the start of the daily period having the lowest mean total insulin delivery and beginning the daytime segment at an offset comprising the daily period from the beginning of the overnight segment.

Example 16 is an extension of Example 15, or any other example disclosed herein, wherein the mean total insulin delivery is determined based on insulin delivered by an automatic drug delivery system.

Example 17 is an extension of Example 15, or any other example disclosed herein, wherein the daily period is 8 hours and the predetermined past number of days is 7 days, such that the mean total insulin delivery is determined for any 8 hour daily period over the last 7 days.

Example 18 is an extension of Example 15, or any other example disclosed herein, wherein the mean total insulin delivery is calculated for 8 hour daily periods beginning every 5 minutes.

Example 19 is an extension of Example 15, or any other example disclosed herein, the method further comprising determining a quantity of long-acting insulin to be administered during the overnight segment and determining a quantity of long-acting insulin to be administered during the daytime segment.

Example 20 is extension of Example 19, or any other example disclosed herein, wherein determining the quantity of long-acting insulin to be administered during the overnight segment comprises determining an average insulin delivery during the daily period comprising the overnight segment over the predetermined past number of days.

Example 21 is an extension of Example 20, or any other example disclosed herein, wherein determining the quantity of long-acting insulin to be administered during the daytime segment comprises determining an average insulin delivery during the daytime segment over the predetermined past number of days.

Example 22 is an extension of Example 21, or any other example disclosed herein, wherein the average insulin delivery for the overnight segment and the daytime segment are expressed in units per hour of insulin.

Example 23 is an extension of Example 22, or any other example disclosed herein, wherein the average insulin delivery is determined based on insulin delivered by an automatic drug delivery system.

Example 24 is an extension of example 23, or any other example disclosed herein, the method further comprising providing the basal profile to the automatic drug delivery system for use in a manual mode of insulin delivery.

Example 25 is an extension of example 24, or any other example disclosed herein, wherein the basal profile is visualized in a user interface of the automatic drug delivery system.

Example 26 is a fourth embodiment of the invention comprising a method of adjusting a basal profile of a user of an automatic drug delivery system comprising calculating a unitless term representing a mean quality of glucose control for the user during any segment of the basal profile for hyperglycemic incidence over past predetermined period of time, calculating unitless term representing a mean quality of glucose control for user during any segment of the basal profile for hypoglycemic incidence over the past predetermined period of time and calculating a new rate of insulin delivery for the segment based on the unitless terms.

Example 27 is an extension of Example 26, or any other example disclosed herein, wherein the past predetermined period of time is 7 days.

Example 28 is an extension of Example 26, or any other example disclosed herein, wherein the rate of insulin delivery for the segment is lowered if the user experienced the hypoglycemic condition during the entire segment.

Example 29 is an extension of Example 28, or any other example disclosed herein, wherein the rate of insulin delivery for the segment is raised if the user experienced a hyperglycemic condition during the entire segment.

Example 30 is an extension of Example 29, or any other example disclosed herein, wherein a hypoglycemic condition exists if blood glucose readings of the user were below 40 mg/dL during the entire segment.

Example 31 is an extension of Example 30, or any other example disclosed herein, wherein a hyperglycemic condition exists if blood glucose readings of the user were above 400 mg/dL during the entire segment.

Example 32 is an extension of Example 27, or any other example disclosed herein, wherein the rate of insulin delivery for the segment is lowered if the user experienced any hypoglycemic incidence during the segment over the past predetermined period of time.

Example 33 is an extension of Example 32, or any other example disclosed herein, wherein the rate of insulin delivery for the segment is raised of the user experienced any hyperglycemic incidence during the segment over the past predetermined period of time.

Example 34 is an extension of Example 26, or any other example disclosed herein, wherein the insulin delivery rate for the segment can be lowered or raised within the predetermined limit.

Example 35 is an extension of Example 34, or any other example disclosed herein, wherein the predetermined limit is 20%.

Example 36 is an extension of Example 26 or any other example disclosed herein, wherein the adjusted basal profile is provided to the automatic drug delivery system for use in manual mode.

Example 37 is an extension of Example 26, or any other example disclosed herein, wherein the adjusted basal profile is visualized by a user interface of the automatic drug delivery system.

Software related implementations of the techniques described herein may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

To those skilled in the art to which the invention relates, many modifications and adaptations of the invention may be realized. Implementations provided herein, including sizes, shapes, ratings compositions and specifications of various components or arrangements of components, and descriptions of specific manufacturing processes, should be considered exemplary only and are not meant to limit the invention in any way. As one of skill in the art would realize, many variations on implementations discussed herein which fall within the scope of the invention are possible. Moreover, it is to be understood that the features of the various embodiments described herein were not mutually exclusive and can exist in various combinations and permutations, even if such combinations or permutations were not made express herein, without departing from the spirit and scope of the invention. Accordingly, the method and apparatus disclosed herein are not to be taken as limitations on the invention but as an illustration thereof. The scope of the invention is defined by the claims which follow.

Although the present invention is defined in the attached claims, it should be understood that the present invention can also (alternatively) be defined in accordance with the following embodiments:

1. A method of providing an initial basal profile for a user comprising:

   receiving a set of parameters reflecting a multiple daily injection therapy for the user; and

   determining two or more segments for the basal profile, each segment comprising a start time, an end time and a delivery rate, based on the parameters.

2. The method of embodiment 1 wherein the parameters reflect intake of both long-acting and short-acting insulin during multiple daily injection therapy.

3. The method according to any one of embodiments 1 or 2 wherein the basal profile comprises two segments, one segment for periods of least meal activity and one for periods of most meal activity.

4. The method according to any one of embodiments 1 to 3 further comprising:

calculating a mean time for the user's intake of short-acting insulin during multiple daily injection therapy; and

determining a start time for the segment for periods of least meal activity as a time 12 hours after the mean time.

5. The method according to any one of embodiments 1 to 4 further comprising:
determining a start time for the segment for periods of most meal activity as an offset from the start time of the segment for periods of least meal activity.

6. The method according to any one of embodiments 1 to 5 wherein the offset varies between 6 and 10 hours.

7. The method according to any one of embodiments 1 to 6 further comprising:

calculating a basal rate for the segment for periods of least meal activity; and

calculating a basal rate for the segment for periods of most meal activity;

wherein the basal rates are calculated by:

determining an hourly quantity of long-acting insulin based on the parameters;

reducing the hourly quantity by a predetermined percentage for the segment for periods of least meal activity; and

increasing the hourly quantity by a predetermined percentage for the segment for periods of most meal activity.

8. The method according to any one of embodiments 1 to 7 wherein the hourly quantity of long-acting insulin is reduced by a reduction factor reflecting a reduced insulin need when using the automatic drug delivery system.

9. The method according to any one of embodiments 1 to 8 wherein the reduction factor varies between 0.8 and 0.9.

10. The method according to any one of embodiments 1 to 9 wherein the reduced quantity for the segment for periods of least meal activity is reduced by approximately 20% and wherein the increased quantity for the segment for periods of most meal activity is increased by approximately 10%.

11. The method according to any one of embodiments 1 to 10 further comprising:
providing the basal profile to an automatic drug delivery system for use in manual mode.

12. The method according to any one of embodiments 1 to 11 further comprising:
visualizing the basal profile in a user interface of the automatic drug delivery system.

13. A method of providing an initial total daily insulin input for an automatic drug delivery system operating in automatic mode comprising:

receiving a set of parameters reflecting a multiple daily injection therapy for the user;

calculating the total daily insulin administered to the user based on the parameters;

reducing the total daily insulin by a reduction factor reflecting a reduced insulin need when using the automatic drug delivery system;

providing the adjusted total daily insulin to the automatic drug delivery system as a starting basis for operation in automatic mode.

14. The method of embodiment 13 wherein the reduction factor varies between 0.8 and 0.9.

15. A method of providing a basal profile for a user comprising an overnight segment and a daytime segment, the method comprising:

determining a mean total insulin delivery for any daily period over a predetermined past number of days;

determining the daily period having the lowest mean total insulin delivery;

beginning the overnight segment at the start of the daily period having the lowest mean total insulin delivery;

beginning the daytime segment at an offset comprising the daily period from the beginning of the overnight segment.

16. The method of embodiment 15 wherein the mean total insulin delivery is determined based on insulin delivered by an automatic drug delivery system.

17. The method according to any one of embodiments 15 or 16 wherein the daily period is 8 hours and the predetermined past number of days is 7 days, such that the mean total insulin delivery is determined for any 8 hour daily period over the last 7 days.

18. The method according to any one of embodiments 15 to 17 wherein the mean total insulin delivery is calculated for 8 hour daily periods beginning every five minutes.

19. The method according to any one of embodiments 15 to 18 further comprising:

determining a quantity of long-acting insulin to be administered during the overnight segment; and

determining a quantity of long-acting insulin to be administered during the daytime segment.

20. The method according to any one of embodiments 15 to 19 wherein determining the quantity of long-acting insulin to be administered during the overnight segment comprises:
determining an average insulin delivery during the daily period comprising the overnight segment over the predetermined past number of days.

21. The method according to any one of embodiments 15 to 20 wherein determining the quantity of long-acting insulin to be administered during the daytime segment comprises:
determining an average insulin delivery during the daytime segment over the predetermined past number of days.

22. The method according to any one of embodiments 15 to 21 wherein the average insulin delivery for the overnight segment and the daytime segment are expressed in units per hour of insulin.

23. The method according to any one of embodiments 15 to 22 wherein the average insulin delivery is determined based on insulin delivered by an automatic drug delivery system.

24. The method according to any one of embodiments 15 to 23 further comprising:
providing the basal profile to the automatic drug delivery system for use in a manual mode of insulin delivery.

25. The method according to any one of embodiments 15 to 24 wherein the basal profile is visualized in a user interface of the automatic drug delivery system.

26. A method for adjusting a basal profile of a user of an automatic drug delivery system comprising:

calculating a unitless term representing a mean quality of glucose control for the user during any segment of the basal profile for hyperglycemic incidence over a past predetermined period of time;

calculating a unitless term representing a mean quality of glucose control for a user during the segment of the basal profile for hypoglycemic incidence over the past predetermined period of time; and

calculating a new rate of insulin delivery for the segment based on the unitless terms.

27. The method of embodiment 26 wherein the past predetermined period of time is 7 days.

28. The method according to any one of embodiments 26 or 27 wherein the rate of insulin delivery for the segment is lowered if the user experienced a hypoglycemic condition during the entire segment.

29. The method according to any one of embodiments 26 to 28 wherein the rate of insulin delivery for the segment is raised if the user experienced a hyperglycemic condition during the entire segment.

30. The method according to any one of embodiments 26 to 29 wherein a hypoglycemic condition exists if blood glucose readings of the user were below 40 mg/dL during the entire segment over the past predetermined period of time.

31. The method according to any one of embodiments 26 to 30 wherein a hyperglycemic condition exists if blood glucose readings of the user were above 400 mg/dL during the entire segment.

32. The method according to any one of embodiments 26 to 31 wherein the rate of insulin delivery for the segment is lowered if the user experiences any hypoglycemic incidence during the segment over the past predetermined period of time.

33. The method according to any one of embodiments 26 to 32 wherein the rate of insulin delivery for the segment is raised if the user experiences any hyperglycemic incidence during the segment over the past predetermined period of time.

34. The method according to any one of embodiments 26 to 33 wherein the insulin delivery rate for the segment can be lowered or raised within a predetermined limit.

35. The method according to any one of embodiments 26 to 34 wherein the predetermined limit is 20%.

36. The method according to any one of embodiments 26 to 35 wherein the adjusted basal profile is provided to the automatic drug delivery system for use in manual mode.

37. The method according to any one of embodiments 26 to 36 wherein the adjusted basal profile is visualized in a user interface of the automatic drug delivery system.

**Claims**

1. A method of providing an initial basal profile for a user comprising:

   receiving a set of parameters reflecting a multiple daily injection therapy for the user; and
   determining two or more segments for the basal profile, each segment comprising a start time, an end time and a delivery rate, based on the parameters.

2. The method of claim 1 wherein the parameters reflect intake of both long-acting and short-acting insulin during multiple daily injection therapy.

3. The method according to any one of claims 1 or 2 wherein the basal profile comprises two segments, one segment for periods of least meal activity and one for periods of most meal activity.

4. The method according to any one of claims 1 to 3 further comprising:

   calculating a mean time for the user's intake of short-acting insulin during multiple daily injection therapy; and
   determining a start time for the segment for periods of least meal activity as a time 12 hours after the mean time.

5. The method according to any one of claims 1 to 4 further comprising:
   determining a start time for the segment for periods of most meal activity as an offset from the start time of the segment for periods of least meal activity.

**6.** The method according to any one of claims 1 to 5 wherein the offset varies between 6 and 10 hours.

**7.** The method according to any one of claims 1 to 6 further comprising:

calculating a basal rate for the segment for periods of least meal activity; and
calculating a basal rate for the segment for periods of most meal activity;
wherein the basal rates are calculated by:

determining an hourly quantity of long-acting insulin based on the parameters;
reducing the hourly quantity by a predetermined percentage for the segment for periods of least meal activity; and
increasing the hourly quantity by a predetermined percentage for the segment for periods of most meal activity.

**8.** The method according to any one of claims 1 to 7 wherein the hourly quantity of long-acting insulin is reduced by a reduction factor reflecting a reduced insulin need when using the automatic drug delivery system.

**9.** The method according to any one of claims 1 to 8 wherein the reduction factor varies between 0.8 and 0. 9.

**10.** The method according to any one of claims 1 to 9 wherein the reduced quantity for the segment for periods of least meal activity is reduced by approximately 20% and wherein the increased quantity for the segment for periods of most meal activity is increased by approximately 10%.

**11.** The method according to any one of claims 1 to 10 further comprising:
providing the basal profile to an automatic drug delivery system for use in manual mode.

**12.** The method according to any one of claims 1 to 11 further comprising:
visualizing the basal profile in a user interface of the automatic drug delivery system.

**13.** A method of providing an initial total daily insulin input for an automatic drug delivery system operating in automatic mode comprising:

receiving a set of parameters reflecting a multiple daily injection therapy for the user;
calculating the total daily insulin administered to the user based on the parameters;
reducing the total daily insulin by a reduction factor reflecting a reduced insulin need when using the automatic drug delivery system;
providing the adjusted total daily insulin to the automatic drug delivery system as a starting basis for operation in automatic mode.

**14.** The method of claim 13 wherein the reduction factor varies between 0.8 and 0.9.

**15.** A method of providing a basal profile for a user comprising an overnight segment and a daytime segment, the method comprising:

determining a mean total insulin delivery for any daily period over a predetermined past number of days;
determining the daily period having the lowest mean total insulin delivery;
beginning the overnight segment at the start of the daily period having the lowest mean total insulin delivery;
beginning the daytime segment at an offset comprising the daily period from the beginning of the overnight segment.

FIG. 1

Receive Current MDI
Parameters
202

Calculate TDI based on MDI
Parameters
204

Calculate Segment N Start
Time, End Time and Rate
208

Provide TDI to ADD System for
Use in Automatic Mode
206

No

Desired Number of
Segments Calculated?
210

Yes

Provide Basal Profile to ADD
System for Use in Manual
Mode
212

*FIG. 2*

Calculate Mean Delivery for
Multiple 8 Hour Periods in the
Previous 7 Days
302

Calculate Overnight Segment
Start Time, End Time and
Quantity
304

Calculate Daytime Segment
Start Time, End Time and
Quantity
306

Output Basal Profile
308

*FIG. 3*

Calculate Mean Quality of
Hyperglycemic Incidence
402

Calculate Mean Quality of
Hypoglycemic Incidence
404

Calculate Adjustment to Basal
Segment When Hypo or Hyper
For Full Segment Duration
406a

Calculate Adjustment to Basal
Segment When any Hypo
Experienced During Segment
406b

Adjust Basal Segment
408

*FIG. 4*

**Basal Profile A**

| Start | End | Quantity |
|---|---|---|
| 00:00 | 08:00 | 0.4 U/h |
| 08:00 | 14:00 | 0.6 U/h |
| 14:00 | 18:00 | 0.9 U/h |
| 18:00 | 20:00 | 0.7 U/h |
| 20:00 | 24:00 | 0.6 U/h |

**Basal Profile A**

| Start | End | Quantity |
|---|---|---|
| 00:00 | 08:00 | 0.4 U/h |
| 08:00 | 14:00 | 0.5 U/h* |
| 14:00 | 18:00 | 0.9 U/h |
| 18:00 | 20:00 | 0.8 U/h* |
| 20:00 | 24:00 | 0.6 U/h |

*Profiles Modified; Tap to see Original Values

**Basal Profile A**

| Start | End | Quantity |
|---|---|---|
| 00:00 | 08:00 | 0.4 U/h |
| 08:00 | 14:00 | 0.5 U/h |
| 14:00 | 18:00 | 0.9 U/h |
| 18:00 | 20:00 | 0.8 U/h |
| 20:00 | 24:00 | 0.6 U/h |

Accept Modified Profiles

**Basal Profile A**

| Start | End | Quantity |
|---|---|---|
| 00:00 | 08:00 | 0.4 U/h |
| 08:00 | 14:00 | 0.6 U/h* |
| 14:00 | 18:00 | 0.9 U/h |
| 18:00 | 20:00 | 0.7 U/h* |
| 20:00 | 24:00 | 0.6 U/h |

*: we recommend increasing this profile by 0.1U/h
*: we recommend reducing this profile by 0.1U/h

*FIG. 5A*

502

**Basal Profiles**
Your Basal Profiles

**Basal Profile A**
Currently Active

Basal Profile B

Basal Profile C

Activate Automated Profile

510

508

**Basal Profile A** ⟋504

| Start | End | Quantity |
|---|---|---|
| 00:00 | 08:00 | 0.4 U/h |
| 08:00 | 14:00 | 0.6 U/h |
| 14:00 | 18:00 | 0.9 U/h |
| 18:00 | 20:00 | 0.7 U/h |
| 20:00 | 24:00 | 0.6 U/h |

**Automated Profile**

| Start | End | Quantity |
|---|---|---|
| 00:00 | 08:00 | 0.4 U/h |
| 08:00 | 14:00 | 0.5 U/h |
| 14:00 | 18:00 | 0.9 U/h |
| 18:00 | 20:00 | 0.8 U/h |
| 20:00 | 24:00 | 0.6 U/h |

506

*FIG. 5B*

512

**Basal Profile A**

**Basal Profile Change Recommended**
The system recommends the following changes to this basal profile:
08:00~14:00 0.5→0.6U/h
08:00~14:00 0.5→0.6U/h

Accept    Cancel

516

514

**Basal Profile A**

| Start | End | Quantity |
|---|---|---|
| 00:00 | 08:00 | 0.4 U/h |
| 08:00 | 14:00 | 0.5 U/h |
| 14:00 | 18:00 | 0.9 U/h |
| 18:00 | 20:00 | 0.8 U/h |
| 20:00 | 24:00 | 0.6 U/h |

*FIG. 5C*

Europäisches
Patentamt

European
Patent Office

Office européen
des brevets

# EUROPEAN SEARCH REPORT

Application Number

EP 23 16 7779

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/213200 A1 (RASKIN EDWARD B [US] ET AL) 15 July 2021 (2021-07-15) * paragraph [0069] - paragraph [0074] * * paragraph [0129] - paragraph [0192] * * paragraph [0319] - paragraph [0342] * * figures 2, 7-18 * | 1-15 | INV. G16H20/17 A61M5/14 G16H40/67 G16H50/20 |
| A | GEORGE GRUNBERGER ET AL: "Consensus Statement by the American Association of Clinical Endocrinologists/American College of Endocrinology Insulin Pump Management Task Force", ENDOCRINE PRACTICE, vol. 20, no. 5, 1 May 2014 (2014-05-01), pages 463-489, XP055425648, US ISSN: 1530-891X, DOI: 10.4158/EP14145.PS * the whole document * | 1-15 | |
| A | US 2021/050085 A1 (HAYTER GARY A [US] ET AL) 18 February 2021 (2021-02-18) * figures 1-18B * | 1-15 | **TECHNICAL FIELDS SEARCHED (IPC)** |
| A | US 10 957 438 B2 (ASEKO INC [US]) 23 March 2021 (2021-03-23) * figures 1-12 * | 1-15 | G16H A61M |

The present search report has been drawn up for all claims

| Place of search | Date of completion of the search | Examiner |
|---|---|---|
| Munich | 16 August 2023 | Schechner-Resom, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 7779

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2023

| Patent document cited in search report | | Publication date | Patent family member(s) | | Publication date |
|---|---|---|---|---|---|
| US 2021213200 | A1 | 15-07-2021 | NONE | | |
| US 2021050085 | A1 | 18-02-2021 | AU | 2020324387 A1 | 10-02-2022 |
| | | | CA | 3147267 A1 | 11-02-2021 |
| | | | CN | 114206207 A | 18-03-2022 |
| | | | EP | 4007523 A1 | 08-06-2022 |
| | | | JP | 2022543239 A | 11-10-2022 |
| | | | US | 2021050085 A1 | 18-02-2021 |
| | | | WO | 2021026004 A1 | 11-02-2021 |
| US 10957438 | B2 | 23-03-2021 | US | 2017351842 A1 | 07-12-2017 |
| | | | US | 2019074078 A1 | 07-03-2019 |
| | | | WO | 2017213933 A1 | 14-12-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7303549 B
- US 7137964 B

- US 6740059 B